# EUROPEAN PATENT SPECIFICATION

(11) **EP 1 447 445 B1**
(45) Date of publication and mention of the grant of the patent: **14.03.2007**
(21) Application number: 02778043.6
(22) Date of filing: 01.11.2002
(51) Int. Cl.: C12N 15/09, C12Q 1/68, G01N 33/53, G01N 33/566, B01J 19/00

(54) **PROBE SOLUTION COMPOSITIONS, REACTIVE CHIP USING THE SAME AND PROCESS FOR THE PRODUCTION THEREOF**
ZUSAMMENSETZUNGEN FÜR LÖSUNGEN FÜR SONDEN, REAKTIONSFÄHIGER CHIP BEI DEM DIESE VERWENDET WERDEN, SOWIE HERSTELLUNGSVERFAHREN
COMPOSITIONS DE SOLUTION DE SONDE, PUCE REACTIVE FAISANT APPEL AUXDITES COMPOSITIONS ET SON PROCEDE DE PRODUCTION

(30) Priority: 02.11.2001 JP 2001338490
(43) Date of publication of application: 18.08.2004
(73) Proprietor: NGK INSULATORS, LTD., Nagoya-shi, Aichi 467-8530 (JP)
(72) Inventor: YOSHIDA, Yasuko, Nagoya-shi, Aichi 454-0943 (JP); TANAKA, Naoko, Yokkaichi-shi, Mie 510-8028 (JP); YAMADA, Saichi, Ichinomiya-shi, Aichi 491-0015 (JP)
(74) Representative: Paget, Hugh Charles Edward
(86) International application number: PCT/JP2002/011454
(87) International publication number: WO 2003/038089

(56) References cited:
- EP-A1- 1 026 259
- EP-A2- 1 093 855
- WO-A-00/23023
- JP-A- 2001 116 750

## Description

### Technical Field

The present invention relates to a probe solution composition employed for producing a reactive chip in which a probe capable of binding specifically to a target substance is aligned and fixed at a high density on a substrate, a reactive chip produced by using this composition, as well as a method for producing this reactive chip.

### Background Art

For the purpose of rapidly analyzing a gene structure or gene expression mode on a large scale, various reactive chips are employed. Such a reactive chip has several-thousand to several ten-thousand types or more of different probes which are aligned and fixed as spots on a substrate such as a glass slide, and can specify a target substance or deterimine quantity of the target substance in a sample using as an index the presence or absence of the binding of the target substance labeled for example with a fluorescence to the probe. The probe to be fixed on a chip may vary depending on the type of the target substance to be analyzed. For example, when using DNA or RNA as a target substance, a probe capable of binding complementarily (hybridizing) therewith, such as a double-stranded or single-stranded DNA fragment or polynucleotide chain or oligonucleotide chain, is employed so that the chip is called DNA chip (or DNA array). The spotting of a probe, when using a DNA fragment as a probe, is effected by a method in which the DNA fragment itself is fixed on a substrate or a method in which a DNA fragment having a certain base sequence is synthesized on the substrate. In any method, a fixed amount of the respective probe material should be spotted accurately on a predetermined position.

A known method for spotting a probe material on a substrate is a method in which a pin is used to supply (shot) a probe solution onto the substrate, such as a QUILL method, pin and ring method, spring pin method and the like.

Unlike such a method, the applicants of this application invented a method for spotting an extremely small amount of a probe solution rapidly and accurately on a certain position on a substrate by means of using a jet nozzle, and applied for the patent for this technology (JP-A-2001-116750). The applicants of this application also applied for the patent for a method for producing a DNA chip allowing the probe solution efficiency to be increased and allowing the spot diameter to be uniform by means of spotting the DNA probe solution plural times repetitively (repetitive shot spotting) (JP-A-2001-186880).

On the other hand, a probe solution contains carboxymethyl cellulose and the like for the purpose of fixing a probe spot on a substrate stably, and the composition of the probe solution has also been improved for obtaining a further higher detection sensitivity and a further higher spot density. For example, JP-A-2000-295990 discloses the probe solution (DNA solution) containing a hydrophilic polymer. JP-A-2001-66305 discloses, as a probe solution in an ink jet method, the liquid composition consisting of a probe, urea, glycerin, thiodiglycol and acetylene alcohol.

In this post-genome generation requiring a rapid identification of a very large number of unknown genes and an analysis of functions thereof, DNA chips and other reactive chips are demanded increasingly to serve as a hopeful means. In response to such a demand, there exists problems still to be solved before accomplishing an inexpensive supply and an efficient mass production of a reactive chip capable of identifying and quantifying a target substance at a high accuracy. One problem is related to a further improbement in the detection sensitivity which allows an extremely small amount of a target substance to be measured, for which a further stabilization of the spot shape is required. Such a further stabilization of the spot shape enables a further higher density of the probe spots and allows different biological molecular probes to be spotted under an uniform condition. Nevertheless, a conventional probe solution composition poses a limitation in obtaining a satisfactory spot shape stably.

On the other hand, a conventional probe solution composition usually employs a salt-containing buffer such as an SSC buffer, sodium carbonate buffer , sodium acetate buffer and the like for increasing the viscosity of the probe solution. Also in the case of fixing a probe via a covalent bond between an epoxy-coated substrate and a modified amino group on the probe, a salt-containing buffer (especially SSC buffer and the like) is employed preferably for the purpose of increasing the efficiency of the covalent bond. However, the use of such a salt-containing buffer may pose a problem of a poor spot shape due to the salt precipitation.

Furthermore,: an ordinary reactive chip manufacturing process requires, after the spotting, a water addition step for preventing the spot from drying as well as a baking step and a fixation step. The omission of those steps is an objective to be accomplished for the purpose of a mass production of a reactive chip at a low cost. Especially in the repetitive shot spotting disclosed in JP-A-2001-186880, the omission of the steps is expected to contribute greatly to the increase in the production efficiency and the reduction in the cost. Nevertheless, a conventional method for producing a reactive chip or a modification of the composition of a probe sample solution is not successful in solving the problems described above entirely.

The invention is established under the circumstance discussed above, and its objective is to provide a novel probe solution composition which enables all of an improvement in the detection sensitivity, stabilization of the spot shape and simplification of the manufacturing process.

Another objective of the invention is to provide a reactive chip produced by using the composition described above as well as a method for producing said reactive chip.

### Disclosure of The Invention

Thus, the invention provides a probe solution composition as set out in claim 1.

In a preferred embodiment of this probe solution, at least 10⁻³ mol/l of salt is further contained.

The invention also provides a reactive chip comprising the probe solution composition as a spot fixed on a substrate.

The invention further provides a method for producing a reactive chip comprising spotting the probe solution composition on a substrate and fixing this liquid composition spot on the substrate.

In the preferred embodiments of this production method, the spotting is effected by an ink jet method, the spot of the probe solution composition is fixed without adding water, and the spotting is repeated twice or more to fix the probe solution composition in a form of a laminate.

### Brief Description of The Drawings

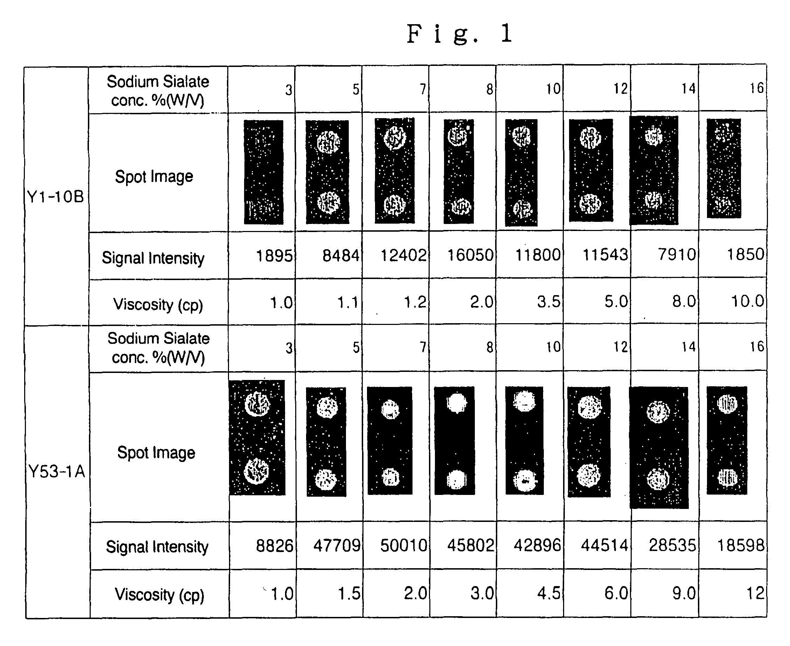

Figure 1 shows the results of the measurement of the signal intensity in a DNA chip prepared using respective probe solution compositions containing an aqueous solution of sodium sialate at various concentrations.

### Best Mode for Carrying Out The Invention

The invention provides a probe solution composition as set out in claim 1.

The moisturizer may for example be a substance capable of retaining residual water of 90% or more when allowed to stand for 10 minutes at 25°C and 40% relative humidity after dropping 1 ml of an aqueous solution containing it onto a filter paper, in comprison to the initially dropped amount. Such a moisturizer may have a molecular weight of 300 to 2000 and a viscosity of 1 to 20 cps. Specifically in the probe solution composition of the invention, there is used one or more of sialic acid, sialic acid salt, sialic acid oligomer, sialic acid oligomer salt, colominic acid and colominic acid salt (hereinafter referred to as "sialic acid analogues" collectively). A sialic acid (salt) oligomer is a dimer to hexamer of sialic acid (salt), and a colominic acid (salt) is heptamer of sialic acid (salt) or higher oligomer. Any of these sialic acid analogues may be any commercial product such as a dried product.

The probe employed in the solution composition of the invention is a biological molecule which binds specifically to a target substance. For example when the target substance is a DNA fragment derived from a genome DNA (for example cDNA), the probe is one which hybridizes with said DNA fragment on the basis of the complementarity, such as a single-stranded DNA fragment, RNA fragment, nucleotide chain (polynucleotide of 100 bases or more, or oligonucleotide of less than 100 bases) and the like. When the target substance is a protein, the probe is one which binds specifically to partial the amino acid sequence of said protein, such as a protein or peptide. It is also possible to use as a probe an antibody capable of binding to an epitope of a protein as well as its Fab, F(ab')₂, Fv fragments and the like.

The concentration of the probe in this probe solution composition may vary depending on the type and the molecular weight of the probe, and it may be 0.1 to about 5 µg in the case for example of a DNA fragment or nucleotide chain. The concentration of the moisturizer may vary depending on the moisturizing ability or viscosity thereof, and it may be 1 to 15 % by weight in the case of a sialic acid analogue.

The constitutents of the solution composition other than the probe and the moisturizer may be similar to those of a probe solution employed in the production of a known reactive chip. For example, a probe and a moisturizer may be mixed with a buffer at pH7 to 8 to prepare the probe solution composition. While the buffer may be a salt-free buffer, it is preferably a salt-containing buffer such as an SSC buffer, sodium carbonate buffer , sodium acetate buffer and the like. A salt-free buffer may employ hydrochloride, hydrobromide, sulfate, nitrate, acetate, benzoate, maleate, fumarate, succinate, tartarate, citrate, oxalate, methanesulfonate, toluenesulfonate, aspartate, glutamate and the like. The concentration of salt may be at least 10⁻³ mol/l, preferably 10⁻² to 1 mol/l as revealed in EXPERIMENTS described below.

A typical example of an inventive probe solution composition, when employing a DNA fragment as a probe, contains an aqueous solution of sodium sialate and the DNA fragment dissolved at 10% (w/v) and 0.01% (w/v), respectively, as final concentration in TE buffer (pH8, 0.01M Tris-HCl, 0.001M EDTA).

As shown in the Examples, a solution containing a sialic acid and a solution containing a probe may be prepared separately, and then mixed with each other to prepare the probe solution composition. In such a case, by providing a large amount of a solution of a sialic acid analogue and mixing with plural types of the probe solutions separately, all types of the probe solution compositions to be spotted onto a reactive chip can efficiently be prepared.

A reactive chip of the invention and a method for producing it are discussed below. The reactive chip is characterized in which the probe solution composition described above has been fixed as a spot on a substrate. The substrate may be a glass slide employed in an ordinary reactive chip, and the poly L-lysine-coated substrate disclosed in JP-A-2001-186880 is preferred especially. This substrate allows the probe to be fixed on the substrate via an electrostatic bond between the negative charge on a DNA itself and the positive charge on an amino acid. When using an epoxy-coated substrate, it is preferred to use the probe solution composition containing salt.

The probe solution composition to be spotted may for example be a solution composition containing 100 to 20000 types of different probes, and the spotted composition forms the spots each having a diameter of 50 to 500 µm and are located at a distance of about 100 to 1000 µm from each other.

A spotting method for producing such a reactive chip may be a conventional pin method, but the ink jet method disclosed in JP-A-2001-116750 and JP-A-2001-186881 is employed preferably. A probe spotted by these methods can be fixed in satisfactory form on a substrate with the aid of the effect of a moisturizer. Also when using a salt-containing buffer, a precipitation of salt is suppressed and a satisfactory spot shape can be obtained. Then such a satisfactory spot shape leads to an increased detection sensitivity and a higher spot density.

After the spotting, each spot is fixed on a substrate by a procedure similar to that for an ordinary reactive chip production which involves cooling, addition of water to the spots (keeping at a humidity of about 80% over a certain time period), fixation by sintering and drying, and the like. Nevertheless, the water addition step described above may be omitted in the method without undergoing any deterioration of the spot shape (such as a doughnut-like spot), since the spotted probe solution composition has a moisture-keeping ability.

Also in the production method of the invention, a repetitive shot spotting disclosed in JP-A-2001-186880 is a preferred embodiment. In such a case, an effect of repetitive shot can further be enhanced since a satisfactory spot shape can be maintained on the basis of each spotting. Also since no water addition is required in the repetitive shot, smaller number of the steps is required for producing a reactive chip.

The invention is further described in the following Examples and Experiments, which are not intended to restrict the invention.

### Examples

### Example 1

### (1) Preparation of liquid compositions

A lyophilized sodium (Na) sialate was dissolved in the constituents shown in Table 1 and the pH was adjusted to prepare a sodium sialate solution at a final concentration ranging from 9 to 48%.

**Table 1**

| | | | | | | | | |
|---|---|---|---|---|---|---|---|---|
| Final concentration of sodium sialate (%(w/v)) | 3 | 5 | 7 | 8 | 10 | 12 | 14 | 16 |
| Adjusted concentration of sodium sialate (%(w/v)) | 9 | 15 | 21 | 24 | 30 | 36 | 42 | 48 |
| Sodium sialate (g) | 2.25 | 3.75 | 5.25 | 6.0 | 7.5 | 9.0 | 10.5 | 12.0 |
| Purified water (ml) | 9.0 | 9.0 | 9.0 | 9.0 | 9.0 | 9.0 | 9.0 | 9.0 |
| 6N NaOH aq (ml) | 2.0 | 2.0 | 2.5 | 2.5 | 3.5 | 4.5 | 5.5 | 6.5 |
| Fill up (ml) | 14.0 | 14.0 | 13.5 | 13.5 | 12.5 | 11.5 | 10.5 | 9.5 |
| pH (final TE: sialic acid: water=1:1:0.8) | 8.05 | 8.03 | 7.95 | 7.98 | 7.97 | 7.92 | 7.98 | 7.98 |

On the other hand, the yeast (Saccharomyces cerevisiae)-derived DNAs (Y1-10B and Y53-1A) were purified, and dissolved each in 200 µl of TE buffer to prepare respective DNA solutions. The DNA concentration of these solutions measured by an absorption photometry was 3 µg/ml.

The DNA solution (20 µl), the sodium sialate solution at each concentration (20 µl) and pure water (20 µl) were mixed to prepare respective liquid composition (DNA/Sodium sialate solution) shown in Table 2.

**Table 2**

| | Final concentration of sodium sialate (%(w/v)) | | | | | | | |
|---|---|---|---|---|---|---|---|---|
| Y1-10B | | | | | | | | |
| (1 µg/ml Aqueous solution of sialic acid) | 3 | 5 | 7 | 8 | 10 | 12 | 14 | 16 |
| 8 Types | | | | | | | | |
| Y53-1A | | | | | | | | |
| (1 µg/ml Aqueous solution of sialic acid) | 3 | 5 | 7 | 8 | 10 | 12 | 14 | 16 |
| 8 Types | | | | | | | | |

### (2) DNA Chip production

Each liquid composition (DNA/Sodium sialate solution) prepared in Step (1) described above was filled in a micropipette fitted with an ink jet nozzle (for example, the device described in JP-A-2001-186881), and 200 pl portion thereof was spotted onto a poly L-lysine-coated glass slide. Then, water was added to each spotted area, which was then sintered at about 80°C for 1 hour to effect the fixation, whereby obtaining 16 types of the DNA chips having DNA probes fixed thereon.

### (3) Measurement

### (3-1) Preparation of labeled cDNA

As a sample for 5 pieces of DNA chips, 5 µl of an yeast mRNA, 1 µl of a DEPC-treated water and 1 µl of an oligo primer were mixed and heated at 70°C for 5 minutes and 42°C for 3 minutes. To this, 4 µl of 5 x reaction buffer, 2 µl of dNTP mixture, 2 µl of 100mM DTT, 2 µl of 1mM FluoroLink dUTP and 2.5 µl of 40 units RNase inhibitor were mixed. To this, 1 µl of Superscript II was added, and the mixture was heated at 42°C for 40 minutes, and then added further with 0.5 µl of the Superscript II, and heated at 42°C for 40 minutes, added with 20 µl of sterilized water, 5 µl of 0.5M EDTA and 10 µl of 1N NaOH, and then heated at 65°C for 60 minutes. The mixture was neutralized with 25 µl of a Tris (1M, pH7.5), filtered with centrifuging at 8000 rpm for 5 minutes using Microcon 30, added with 250 µl of sterilized water, subjected twice to the centrifugation at 8000 rpm for 10 minutes, followed by the centrifugation at 3000 rpm for 45 seconds, whereby recovering the labeled cDNA.

### (3-2) Hybridization

At first, 24.5 µl of the labeled cDNA, 8.75 µl of 20xSSC and 1.75 µl of 10% SDS were mixed, and then the mixture was heated at 95°C for 3 minutes, allowed to stand at room temperature for 15 minutes, spotted twice in a volume of each 17.5 µl per DNA chip. The chip was covered gently with a glass cover while avoiding any residual air bubbles and allowed to incubate in a humid environment at 65°C for 16 hours.

Then, the glass cover was taken off gently in 2xSSC-0.1% SDS, and the chip was washed twice with 2xSSC-0.1% SDS, washed twice with 2xSSC-0.1% SDS heated at 55°C, rinsed with 0.2xSSC-0.1% SDS, 0.2xSSC and 0.05xSSC, centrifuged and dried.

### (3-3) Fluorometry

The DNA chip after the hybridization was set in a Scan Array (BM KIKI Scanner), and measured at Laser Power of 80%, PMT of 80% and resolution degree of 10 µm.

### (3-4) Results

The results are shown in Figure 1. As evident from Figure 1, the DNA chip produced using the probe solution composition containing sodium sialate exhibited a high signal intensity especially at a concentration of 3 to 14%.

### Example 2

### (1) Preparation of liquid compositions

Each of the lyophilized sodium (Na) sialate and dried oligomers (dimer, trimer, tetramer, pentamer, hexamer) thereof was dissolved similarly to Example 1 and the pH was adjusted to prepare a sodium sialate solution whose final concentration was 30%.

On the other hand, the yeast (Saccharomyces cerevisiae)-derived DNAs (Y1-10B and Y53-1A) were purified, and dissolved each in 100 µl of TE buffer to prepare respective DNA solutions. The DNA concentration of each solution measured by an absorption photometry was 3.03 µg/ml.

The DNA solution (20 µl), each sodium sialate solution (20 µl) and pure water (20 µl) were mixed to prepare liquid composition (DNA/Sodium sialate solution) shown in Table 3.

**Table 3**

| No. | Additive | Final additive concentration | Final DNA concentration |
|---|---|---|---|
| | | (%) | (µg/ml) |
| 1 | Sodium sialate aqueous solution | 10 | 1.01 |
| 2 | Sialic acid dimer aqueous solution | 10 | 1.01 |
| 3 | Sialic acid trimer aqueous solution | 10 | 1.01 |
| 4 | Sialic acid tetramer aqueous solution | 10 | 1.01 |
| 5 | Sialic acid pentamer aqueous solution | 10 | 1.01 |
| 6 | Sialic acid hexamer aqueous solution | 10 | 1.01 |
| 7 | Blank (purified water) | - | 1.01 |

### (2) DNA Chip production

Each liquid composition (DNA/Sodium sialate solution) prepared in Step (1) described above was filled in a micropipette fitted with an ink jet nozzle (for example, the device described in JP-A-2001-186881), and a 200 pl portion thereof was spotted onto a poly L-lysine-coated glass slide. Then, water was added to each spotted area, which was then sintered at about 80°C for 1 hour to effect the fixation, whereby obtaining 7 types of the DNA chips having DNA probes fixed thereon.

### (3) Measurement

### (3-1) Preparation of labeled cDNA

As a sample for 5 pieces of DNA chips, 5 µl of an yeast mRNA, 1 µl of DEPC-treated water and 1 µl of an oligo primer were mixed and heated at 70°C for 5 minutes and 42°C for 3 minutes. To this, 4 µl of 5 x reaction buffer, 2 µl of dNTP mixture, 2 µl of 100mM DTT, 2 µl of 1mM FluoroLink dUTP and 2.5 µl of 40 units RNase inhibitor were mixed. To this, 1 µl of Superscript II was added, and the mixture was heated at 42°C for 40 minutes, and then added further with 0.5 µl of the Superscript II, and heated at 42°C for 40 minutes, added with 20 µl of sterilized water, 5 µl of 0.5M EDTA and 10 µl of 1N NaOH, and then heated at 65°C for 60 minutes. The mixture was neutralized with 25 µl of a Tris (1M, pH7.5), filtered with centrifuging at 8000 rpm for 5 minutes using a Microcon 30, combined with 250 µl of sterilized water, subjected twice to the centrifugation at 8000 rpm for 10 minutes, followed by the centrifugation at 3000 rpm for 45 seconds, whereby recovering the labeled cDNA.

### (3-2) Hybridization

At first, 24.5 µl of the labeled cDNA, 8.75 µl of 20xSSC and 1.75 µl of 10% SDS were mixed, and then the mixture was heated at 95°C for 3 minutes, allowed to stand at room temperature for 15 minutes, spotted twice in a volume of each 17.5 µl per DNA chip. The chip was covered gently with a glass cover while avoiding any residual air bubbles and allowed to incubation in a humid environment at 65°C for 16 hours.

Then, the glass cover was taken off gently in 2xSSC-0.1% SDS, and the chip was washed twice with 2xSSC-0.1% SDS, washed twice with 2xSSC-0.1% SDS heated at 55°C, rinsed with 0.2xSSC-0.1% SDS, 0.2xSSC and 0.05xSSC, centrifuged and dried.

### (3-3) Fluorometry

The DNA chip after the hybridization was set in a Scan Array (BM KIKI Scanner), and measured at Laser Power of 80%, PMT of 80% and resolution degree of 10 µm.

### (3-4) Shape evaluation

The shape of a spot containing the DNA probe fixed on the glass was evaluated visually using a stereoscopic microscope and a rate of the satellite appearance was calculated. Also based on the fluorescent image after the hybridization, any satellite which could not be identified visually was identified and a rate of the appearance was calculated.

### (3-5) Results

The results are shown in Table 4. As evident from Table 4, the DNA chip produced using the probe solution composition containing an aqueous solution of a sialic acid analogue exhibited a significantly lower appearance rate of the satellite having poor shape, indicating an ability of providing a satisfactory spot shape in comparison with the DNA chip using a probe solution composition containing pure water.

**Table 4**

| No. | Additive | Stereoscopic microscope evaluation | Hybridization evaluation | total rate of appearance |
|---|---|---|---|---|
| 1 | Sodium sialate aqueous solution | 0.3% | 0.2% | 0.5% |
| 2 | Sialic acid dimer aqueous solution | 0.3% | 0.4% | 0.7% |
| 3 | Sialic acid trimer aqueous solution | 0.4% | 0.3% | 0.7% |
| 4 | Sialic acid tetramer aqueous solution | 0.4% | 0.2% | 0.6% |
| 5 | Sialic acid pentamer aqueous solution | 0.3% | 0.2% | 0.5% |
| 6 | Sialic acid hexamer aqueous solution | 0.2% | 0.4% | 0.6% |
| 7 | Purified water | 15% | 10% | 25% |

### Example 3

### (1) Preparation of liquid compositions

A lyophilized sodium (Na) sialate and a carboxymethyl cellulose (CMC) were dissolved in the constituents shown in Table 5 and the pH was adjusted to prepare a sodium sialate solution at a final concentration ranging from 6 to 42% and CMC solution at 2%.

**Table 5**

| | | | | | | |
|---|---|---|---|---|---|---|
| Final concentration of sodium sialate (%(w/v)) | 2 | 7 | 10 | 14 | Final concentration of CMC (%(w/v)) | 0.7 |
| Adjusted concentration of sodium sialate (%(w/v)) | 6 | 21 | 30 | 42 | Adjusted concentration of CMC | 2 |
| Sodium sialate (g) | 1.5 | 5.25 | 7.5 | 10.5 | CMC (g) | 0.5 |
| Purified water (ml) | 9.0 | 9.0 | 9.0 | 9.0 | Purified water (ml) | 2.5 |
| 6N NaOH aq (ml) | 1.5 | 2.5 | 3.5 | 5.5 | | |
| Fill up (ml) | 14.5 | 13.5 | 12.5 | 10.5 | | |
| pH(final TE: sialic acid: water=1:1:0.8) | 8.05 | 7.95 | 7.97 | 7.98 | pH(final TE:CMC: water=1:1:0.8) | 8.06 |

On the other hand, the yeast (Saccharomyces cerevisiae)-derived DNAs (Y1-10B and Y53-1A) were purified, and dissolved each in 120 µl of TE buffer to prepare respective DNA solutions. The DNA concentration of each solution measured by an absorption photometry was 3.2 µg/ml.

The DNA solution (20 the sodium sialate solution at each concentration (20 µl) and pure water (20 µl) were mixed to prepare a liquid composition (DNA/Sodium sialate solution) in which respective sodium sialate concentration was 2, 7, 10 or 14. The liquid composition in which CMC concentration is 0.5% (DNA/CMC solution) was also prepared.

### (2) DNA Chip production

Each liquid composition prepared in Step (1) described above was filled in a micropipette fitted with an ink jet nozzle (for example, the device described in JP-A-2001-186881), and spotted 200 pl each onto a poly L-lysine-coated glass slide. Then, water was added to each spotted area on the half of the glass slides (3 slides) on each of which each liquid composition had been spotted, which was then sintered at about 80°C for 1 hour to effect the fixation, whereby obtaining the DNA chips having DNA probes fixed thereon. Remaining half of the slides (3 slides) were baked without adding water and fixed to give the DNA chips (Tables 6 and 7).

**Table 6**

| Sialic acid group | Final concentration of sodium sialate (%(W/V)) | | | | | | | |
|---|---|---|---|---|---|---|---|---|
| Y1-10B with 4 concentrations of sialic acid | 2 | | 7 | | 10 | | 14 | |
| With or without water addition | with | without | with | without | with | without | with | without |
| Number of glass slides | 3 | 3 | 3 | 3 | 3 | 3 | 3 | 3 |
| Y53-1A with 4 concentrations of sialic acid | 2 | | 7 | | 10 | | 14 | |
| With or without water addition | with | without | with | without | with | without | with | without |
| Number of glass slides | 3 | 3 | 3 | 3 | 3 | 3 | 3 | 3 |

**Table 7**

| Control-CMC group | Final concentration of CMC(%(w/v)) | |
|---|---|---|
| Y1-10B with 1 concentration of CMC | 0.7 | |
| With or without water addition | with | without |
| Number of glass slides | 3 | 3 |
| Y53-1A with 1 concentration of CMC | 0.7 | |
| With or without water addition | with | without |
| Number of glass slides | 3 | 3 |

### (3) Measurement

### (3-1) Preparation of labeled cDNA

As a sample for 5 pieces of DNA chips, 5 µl of an yeast mRNA, 1 µl of DEPC-treated water and 1 µl of an oligo primer were mixed and heated at 70°C for 5 minutes and 42°C for 3 minutes. To this, 4 µl of 5 x reaction buffer, 2 µl of dNTP mixture, 2 µl of 100mM DTT, 2 µl of 1mM FluoroLink dUTP and 2.5 µl of 40 units RNase inhibitor were mixed. To this, 1 µl of Superscript II was added, and the mixture was heated at 42°C for 40 minutes, and then added further with 0.5 µl of the Superscript II, and heated at 42°C for 40 minutes, added with 20 µl of sterilized water, 5 µl of 0.5M EDTA and 10 µl of 1N NaOH, and then heated at 65°C for 60 minutes. The mixture was neutralized with 25 µl of a Tris (1M, pH7.5), filtered with centrifuging at 8000 rpm for 5 minutes using a Microcon 30, added with 250 µl of sterilized water, subjected twice to the centrifugation at 8000 rpm for 10 minutes, followed by the centrifugation at 3000 rpm for 45 seconds, whereby recovering the labeled cDNA.

### (3-2) Hybridization

At first, 24.5 µl of the labeled cDNA, 8.75 µl of 20xSSC and 1.75 µl of 10% SDS were mixed, and the mixture was heated at 95°C for 3 minutes, allowed to stand at room temperature for 15 minutes, spotted twice in a volume of each 17.5 µl per DNA chip, which was covered gently with a glass cover while avoiding any residual air bubbles and allowed to incubate in a humid environment at 65°C for 16 hours.

Then, the glass cover was taken off gently in 2xSSC-0.1% SDS, and the chip was washed twice with 2xSSC-0.1% SDS, washed twice with 2xSSC-0.1% SDS heated at 55°C, rinsed with 0.2xSSC-0.1% SDS, 0.2xSSC and 0.05xSSC, centrifuged and dried.

### (3-3) Fluorometry-based shape evaluation

The DNA chip after the hybridization was set in a Scan Array (BM KIKI Scanner), and examined for the shape of the fixed spot at Laser Power of 80%, PMT of 80% and resolution degree of 10 µm. Thus, the number of the spots each of which had a shape giving no even brightness within the spot and giving changing fluorescent intensity was counted.

### (3-4) Results

The results are shown in Table 8. When the conventional CMC without adding water poor spots appeared at the probability of 100%. On the contrary, the DNA chip produced using the probe solution composition containing sodium sialate was proben to be capable of preventing the poor spot formation, almost completely at a concentration of 2 to 14%, even without adding water.

**Table 8**

| | | Sodium sialate concentration (%) | | | | | | | | | | | | CMC concentration | |
|---|---|---|---|---|---|---|---|---|---|---|---|---|---|---|---|
| | | | | | | | | | | | | | | (%) | |
| | | 0% | 0% | 2% | 2% | 7% | 7% | 10 % | 10% | 14 % | 14% | 20 % | 20% | 2% | 2% |
| With or without water addition | | with | without | with | Without | with | without | with | without | with | without | with | without | with | without |
| Probability of poor spot appearance (%) | Y1-B10 | 10 | 80 | 5 | 1 | 4 | 0 | 4 | 0 | 5 | 0 | 6 | 3 | 5 | 100 |
| | Y53-1A | 12 | 100 | 7 | 1 | 6 | 0 | 5 | 0 | 5 | 0 | 8 | 4 | 8 | 100 |

### Example 4

### (1) Preparation of liquid compositions

The 20xSSC solution was prepared by which 3M Sodium chloride and 0.3M sodium citrate were mixed and adjusting at pH7.0. Then, sialic acid was dissolved in water to prepare 10% solution of sialic acid. A lyophilized oligo DNA (50-base length) was dissolved in purified water at 100 pmol/µl (1.65 g/µl). Each of these solutions was mixed in the ratio of the DNA solution: 50 pmol/µl, 1xSSC (0.3M sodium chloride and 0.03M sodium citrate) and sialic acid solution: 1%, whereby obtaining a liquid composition (DNA/SSC/sialic acid solution). As a control, a sialic acid-free liquid composition (DNA/SSC) was also prepared.

### (2) DNA Chip production

Each liquid composition (DNA/SSC/sialic acid solution) prepared in Step (1) described above was filled in a micropipette fitted with an ink jet nozzle, and a 200 pl portion thereof was spotted onto an epoxysilane-coated glass slide. Then, the slide was reacted for 8 hours in an incubator at 42°C under 50% humidity to proceed covalently bonding, followed by fixation to obtain a DNA chip having an oligonucleotide DNA probe fixed thereon. The control liquid composition was employed similarly to produce a DNA chip.

### (3) Measurement

### (3-1) Preparation of labeled cDNA

5 µl of a rat mRNA (Clontech, 5 µg/µl), 1 µl of a DEPC-treated water and 1 µl of an oligo (dt) primer were mixed and heated at 70°C for 5 minutes and 42°C for 3 minutes. To this, 4 µl of 5 x reaction buffer, 2 µl of a dNTP mixture, 2 µl of 100mM DTT, 2 µl of mM FluoroLink dUTP and 2.5 µl of 40 units RNase inhibitor were mixed. To this, 1 µl of the reverse transferase (Superscript II) was added, and the mixture was heated at 42°C for 40 minutes, and then added further with 1 µl of the SuperScript II, and heated at 42°C for 40 minutes, added with 20 µl of sterilized water, 5 µl of 0.5M EDTA (pH8) and 25 µl of 1N Tris HCl (pH7.5) to effect neutralization, followed by purification through QIAGEN^{™} column, and the labeled cDNA was recovered using pure water.

### (3-2) Hybridization

At first, 11.25 µl of the labeled cDNA, 8 µl of 20xSSC and 0.7 µl of 10% SDS were mixed, heated at 95°C for 3 minutes, allowed to stand at room temperature for 5 minutes, spotted in a volume of each 15 µl per DNA chip, which was covered gently with a glass cover (24 x 32 mm in size) and allowed to incubate in a humid environment at 65°C for 16 hours.

Then, the glass cover was taken off gently with immersing the DNA chip in 2xSSC-0.1% SDS, and the chip was allowed to stand in 2xSSC-0.1% SDS for 5 minutes, washed with 1xSSC, washed with 0.5xSSC, centrifuged at 900 rpm for 2 minutes and dried.

### (3-3) Fluorometry

The DNA chip after the hybridization was set in Scan Array, and measured at Laser Power of 70%, PMT of 60% and resolution degree of 5 µm.

### (3-4) Results

The results are shown in Figure 9. In comparison with the DNA chip made with using a conventional probe solution composition (DNA/SSC), the DNA chip made with the inventive probe solution composition (DNA/SSC/sialic acid solution) exhibited higher signal intensity for any of the genetic cDNAs measured.

**Table 9**

| Liquid composition | Final concentration | Gene name | | | | |
|---|---|---|---|---|---|---|
| | | Polyubiquitin | α-tubulin | Libosome protein L8 | Myocin heavy chain | GAPDH |
| | | signal intensity | | | | |
| DNA/SSC | DNA: 50 | 25071 | 11976 | 14275 | 31348 | 5302 |
| | (pmol/µl) | | | | | |
| | SSC: 2×10⁻¹ | | | | | |
| | (mol/l) | | | | | |
| DNA/SSC/ sialic acid | DNA: 50 | 30121 | 23270 | 35123 | 50361 | 14272 |
| | (pmol/µl) | | | | | |
| | SSC: 2×10⁻¹ | | | | | |
| | (mol/l) | | | | | |
| | Sialic acid: 1 | | | | | |
| | (%(W/V)) | | | | | |

### Experiment

A preferred range of the concentration of a salt to be contained in a probe solution composition was investigated.

### (1) Methods

Similarly to Example 4 except for changing the salt concentration of the SSC buffer, a solution composition (DNA/SSC/Sialic acid solution) was prepared and used to produce a DNA chip. Also similarly to Example 4, a labeled cDNA was prepared, hybridized and subjected to a fluorometry.

### (2) Results

The results of the detection of myocardial actin cDNA were shown in Table 10. As evident from these results, a high signal intensity can be obtained by containing at least 10⁻³ mol/1, preferably 10⁻² to 1 mol/1.

**Table 10**

| | | | | | | | |
|---|---|---|---|---|---|---|---|
| Final concentration of DNA | 50(pmol/ µl) | | | | | | |
| Final concentration of sialic acid | 1 (%(W/V)) | | | | | | |
| Final concentration of salt (mol/l) (SSC) | 0 | 10⁻³ | 10⁻² | 10⁻¹ | 2×10⁻¹ | 5×10⁻¹ | 1 |
| Signal intensity (gene: myocardial actin) | 700 | 8050 | 25430 | 27520 | 30245 | 21322 | 12342 |

### Industrial Applicability

As detailed above, the invention provides a novel probe solution composition, which is utilized for a fixation of a probe capable of binding specifically to a target substance by means of spotting the probe on a substrate. As a result, an increased detection sensitivity of a reaction chip, a higher spot density and a simplified manufacturing process can be achieved, whereby providing a highly accurate reactive chip at a low cost in a large quantity.

## Claims

1. A probe solution composition containing a probe capable of binding specifically to a target substance and a moisturizer, which is utilized for spotting the probe to be fixed on a substrate,
wherein said probe is DNA fragment, RNA fragment, nucleotide chain, protein, peptide or any mixture thereof,
wherein said moisturizer is one or more selected from sialic acid, sialic acid salt, sialic acid oligomer, sialic acid oligomer salt, colominic acid and colominic acid salt, and the moisturizer content is 1 to 15% by weight.

2. The probe solution composition of claim 1, which further contains at least 10⁻³ mol/l of salt.

3. A reactive chip comprising a spot obtainable by fixing of the probe solution composition of claim 1 or 2 on a substrate.

4. A method for producing a reactive chip comprising spotting the probe solution composition of claim 1 or 2 on a substrate and fixing this liquid composition spot on the substrate.

5. The method according to claim 4, wherein the spotting is effected by an ink jet method.

6. The method according to claim 4 or 5, wherein the spot of the probe solution composition is fixed without adding water.

7. The method according to claim 4 or 6, wherein the spotting is repeated twice or more to fix the probe solution composition in a form of laminate.

## Patentansprüche

1. Sondenlösungszusammensetzung, eine zur spezifischen Bindung an eine Zielsubstanz fähige Sonde und ein Feuchthaltemittel umfassend, die zum Spotten der auf einem Substrat zu fixierenden Sonde eingesetzt wird,
worin die Sonde ein DNA-Fragment, ein RNA-Fragment, eine Nucleotidkette, ein Protein, ein Peptid oder ein beliebiges Gemisch daraus ist,
worin das Feuchthaltemittel eines oder mehrere aus Sialinsäure, Sialinsäuresalz, Sialinsäureoligomer, Sialinsäureoligomersalz, Sialsäure und Sialsäuresalz ist und der Feuchthaltemittelgehalt 1 bis 15 Gew.-% beträgt.

2. Sondenlösungszusammensetzung nach Anspruch 1, die weiters zumindest 10⁻³ mol/l Salz enthält.

3. Reaktiver Chip, einen Spot umfassend, der durch Fixieren einer Sondenlösungszusammensetzung nach Anspruch 1 oder 2 auf einem Substrat erhältlich ist.

4. Verfahren zur Herstellung eines reaktiven Chips, das Spotten einer Sondenlösungszusammensetzung nach Anspruch 1 oder 2 auf ein Substrat und das Fixieren dieses Flüssigkeitszusammensetzungsspots auf dem Substrat umfassend.

5. Verfahren nach Anspruch 4, worin das Spotten mithilfe eines Tintenstrahlverfahrens erfolgt.

6. Verfahren nach Anspruch 4 oder 5, worin der Spot der Sondenlösungszusammensetzung ohne Zusatz von Wasser fixiert wird.

7. Verfahren nach Anspruch 4 oder 6, worin das Spotten zweimal oder öfter wiederholt wird, um die Sondenlösungszusammensetzung in Form eines Laminats zu fixieren.

## Revendications

1. Composition d'une solution de sonde contenant une sonde capable de se lier spécifiquement à une substance cible et à un agent humidifiant, que l'on utilise pour parsemer la sonde à fixer sur un substrat,
où ladite sonde est un fragment d'ADN, un fragment d'ARN, une chaîne de nucléotide, une protéine, un peptide ou tout mélange,
où ledit agent humidifiant est un ou plusieurs sélectionnés parmi acide sialique, sel de l'acide sialique, oligomère de l'acide sialique, sel de l'oligomère de l'acide sialique, acide colominique, et sel de l'acide colominique et la teneur en agent humidifiant est de 1 à 15% en poids.

2. Composition de solution de sonde de la revendication 1, qui contient de plus au moins 10⁻³ mole/l de sel.

3. Puce réactif comprenant un maculage obtenu en fixant une composition d'une solution de sonde de la revendication 1 ou 2 sur un substrat.

4. Méthode de production d'une puce réactive consistant à parsemer la composition de la solution de sonde de la revendication 1 ou 2 sur un substrat et à fixer cette composition liquide sur le substrat.

5. Méthode selon la revendication 4, où le maculage est effectué par une méthode à jet d'encre.

6. Méthode selon la revendication 4 ou 5, où le maculage de la composition de la solution de sonde est fixé sans ajouter d'eau.

7. Méthode selon la revendication 4 ou 6, où le maculage est répété deux fois ou plus pour fixer la composition de la solution de sonde sous une forme de stratification.
